# EUROPÄISCHE PATENTSCHRIFT

(11) **EP 3 573 499 B1**
(45) Veröffentlichungstag und Bekanntmachung des Hinweises auf die Patenterteilung: **08.09.2021**
(21) Anmeldenummer: 18702201.7
(22) Anmeldetag: 25.01.2018
(51) Int. Cl.: A47C 7/72, A61B 5/11, A47C 1/024, A47C 1/032, A47C 20/04

(54) **RUHEMÖBEL MIT EINER WARNEINRICHTUNG UND VERFAHREN ZUM BETREIBEN EINER WARNEINRICHTUNG EINES RUHEMÖBELS**
RECLINING FURNITURE COMPRISING A WARNING DEVICE, AND METHOD FOR OPERATING A WARNING DEVICE OF RECLINING FURNITURE
MEUBLE DE REPOS AVEC UN DISPOSITIF D'AVERTISSEMENT ET PROCÉDÉ POUR LE FONCTIONNEMENT D'UN DISPOSITIF D'AVERTISSEMENT D'UN MEUBLE DE REPOS

(30) Priorität: 27.01.2017 DE 102017101647
(43) Veröffentlichungstag der Anmeldung: 04.12.2019
(73) Patentinhaber: DewertOkin GmbH, 32278 Kirchlengern (DE)
(72) Erfinder: HILLE, Armin, 33659 Bielefeld (DE)
(74) Vertreter: Kleine, Hubertus
(86) Internationale Anmeldenummer: PCT/EP2018/051824
(87) Internationale Veröffentlichungsnummer: WO 2018/138196

(56) Entgegenhaltungen:
- WO-A1-2016/123339
- WO-A1-2016/156779
- DE-A1-102009 010 379
- US-A1- 2003 095 263
- US-A1- 2016 128 610
- US-A1- 2016 354 263

## Beschreibung

Die Erfindung betrifft ein Ruhemöbel mit mindestens einem Sensor und einer mit diesem gekoppelten Warneinrichtung, um bei einem unerwünschten Aufstehen eines Benutzers des Ruhemöbels zu informieren. Die Erfindung betrifft weiterhin ein Verfahren zum Betreiben einer derartigen Warneinrichtung eines derartigen Ruhemöbels.

Insbesondere im Pflege- oder Krankenhausbereich kann es erwünscht sein, dass das Pflegepersonal über ein Aufstehen eines Benutzers des Ruhemöbels informiert wird, beispielsweise um einen möglichen Sturz nach dem Aufstehen verhindern zu können. Als Ruhemöbel sind dabei insbesondere Sitzmöbel wie Ruhesessel, aber auch Liegen oder Betten anzusehen. Relevante Beispiele aus dem Stand der Technik sind WO 2016/156779 A1 und US 2016/128610 A1.

Aus der Druckschrift US 5, 780,798 A ist ein Bett als Ruhemöbel dieser Art bekannt, bei dem unter einer Matratze Sensoren angeordnet sind, die abhängig von einem Gewicht schalten, das auf der Matratze lastet. Die Sensoren sind mit einer Warneinrichtung verbunden, die ein hörbares und/oder sichtbares Alarmsignal abgibt, wenn die Sensoren keine Anwesenheit einer Person im Bett anzeigen, also der Benutzer des Betts dieses verlassen hat.

In vergleichbarer Art ist aus der Druckschrift DE 20 2008 018 080 U1 ein Ruhemöbel mit einem elektromotorischen Möbelantrieb bekannt, bei dem eine Dehnungsmesseinrichtung in den elektromotorischen Möbelantrieb eingesetzt wird, um zu erkennen, ob eine Person das Möbelstück nutzt oder nicht. Wiederum kann über eine Warneinrichtung eine Warnung ausgegeben werden, wenn ein Benutzer oder Patient das Bett verlässt, sodass Pflegepersonal einen möglichen Unfall außerhalb des Ruhemöbels verhindern kann.

Die in den genannten Druckschriften beschriebenen Warneinrichtungen geben ein Warnsignal aus, sobald eine Person das Ruhemöbel bereits ganz oder zumindest teilweise verlassen hat. Auch bei einem teilweisen Verlassen des Ruhemöbels, beispielsweise nach einem Aufrichten, ist jedoch bereits eine nicht unerhebliche Verletzungsgefahr bei manchen Benutzern des Ruhemöbels gegeben. Zudem vergeht Zeit, bis das Pflegepersonal den Standort des Ruhemöbels erreicht hat.

Es ist daher eine Aufgabe der vorliegenden Erfindung, ein Ruhemöbel mit mindestens einem Sensor und einer mit diesem gekoppelten Warneinrichtung und ein Verfahren zum Betreiben einer solchen Warneinrichtung zu schaffen, bei denen möglichst sicher eine Gefährdung eines Benutzers des Ruhemöbels durch Verlassen des Ruhemöbels ausgeschlossen werden kann.

Diese Aufgabe wird durch ein Ruhemöbel bzw. ein Verfahren zum Betreiben einer Warneinrichtung eines derartigen Ruhemöbels mit den Merkmalen des jeweiligen unabhängigen Anspruchs gelöst. Vorteilhafte Ausgestaltungen und Weiterbildungen sind Gegenstand der abhängigen Ansprüche.

Ein erfindungsgemäßes Ruhemöbel zeichnet sich dadurch aus, dass die Warneinrichtung dazu eingerichtet ist, Informationen des mindestens einen Sensors, der am Ruhemöbel angeordnet ist, im Hinblick auf ein Bewegungs- und/oder Verhaltensmuster einer das Ruhemöbel benutzenden Person auszuwerten, um ein Warn- bzw. Informationssignal bereits bei einer Absicht, das Ruhemöbel zu verlassen, auszugeben.

Entsprechend weist ein erfindungsgemäßes Verfahren zum Betreiben einer Warneinrichtung eines derartigen Ruhemöbels die folgenden Schritte auf: Es werden Signale oder Daten des mindestens einen Sensors, der am Ruhemöbel oder auch in einer Umgebung des Ruhemöbels angeordnet ist, erfasst und ausgewertet. Anhand der ausgewerteten Sensorinformationen wird ein Bewegungs- und/oder Verhaltensmuster einer das Ruhemöbel benutzenden Person erstellt. Das Bewegungs- und/oder Verhaltensmuster wird analysiert, um eine Absicht, das Ruhemöbel zu verlassen, zu erkennen. Wird eine derartige Absicht erkannt, wird ein Warn- bzw. Informationssignal ausgegeben.

Anders als beim Stand der Technik wird somit nicht das Resultat eines bereits erfolgten teilweisen oder vollständigen Aufstehens erfasst, sondern Anzeichen, die auf ein bevorstehendes Aufstehen hindeuten. Entsprechend kann ein Warn- bzw. Informationssignal nicht erst dann ausgeben werden, wenn die das Ruhemöbel benutzende Person bereits ganz oder teilweise aufgestanden ist, sondern bereits im Vorfeld. Dieses gibt dem Pflegepersonal mehr Zeit, um den Standort des Ruhemöbels zu erreichen und den Aufstehversuch zu unterbinden bzw. zu begleiten und damit die Person vor möglichen Unfällen zu schützen.

In der Regel ist, insbesondere bei älteren oder pflegebedürftigen Personen ein Aufstehvorgang aus einem Ruhemöbel aus einer entspannt sitzenden oder liegenden Position mit längeren Vorbereitungen verbunden, um den Körper in eine geeignete Position zu bringen, aus der das eigentliche Aufstehen vorgenommen werden kann. Diese Vorbereitungen kündigen sich in einem Bewegungs- und/oder Verhaltensmuster der Person an, die von den Sensoren erfasst werden kann. Beispielsweise erfolgt vor einem Aufstehen ein Bewegungszyklus, bei dem der Körper unter Kraftaufwendung mittels der Arme in Richtung eines Kopfteils (bei einem Bett) oder in Richtung eines geneigten Rückenteils (bei einem Sitzmöbel) bewegt wird.

Ein solcher Bewegungsablauf kann z.B. durch Sensoren, die in einem mittleren Bereich eines Bettes angeordnet sind oder die in Armlehnen eines Sessels angeordnet sind, erkannt werden. Aus Signalen der Sensoren erstellte Bewegungs- und/oder Verhaltensmuster werden dann erfindungsgemäß zur Analyse mit vorgegebenen Vergleichsmustern verglichen. Solche Vergleichsmuster können häufig beobachtbaren Bewegungssequenzen vor dem Aufstehen, mit denen der eigentliche Aufstehvorgang eingeleitet wird, entsprechen. Weiter können patientenspezifische Vergleichsmuster vorgehalten werden, auf die die Warneinrichtung angelernt werden kann. Dazu kann beispielsweise wenigstens ein zuvor erfasstes und erstelltes tatsächliches Bewegungs- und/oder Verhaltensmuster einer Aufsteh-Situation als Vergleichsmuster hinterlegt werden.

Zweckdienlich weist das Ruhemöbel beziehungsweise die Warneinrichtung wenigstens eine Speichereinrichtung auf, in der das besagte Vergleichsmuster in Form wenigstens eines Datensatzes gespeichert und damit hinterlegt werden kann. Ein erster Datensatz ist als werksseitiger Datensatz ausgebildet und korrespondiert zu einem praxiserprobten Vergleichsmuster, welches auch als gemitteltes Vergleichsmuster gemäß dem Bewegungs- beziehungsweise Verhaltensmuster verschiedener Personen entspricht. Bevorzugt kann die Speichereinrichtung mehrere Vergleichsmuster aufnehmen. Ferner können alternativ mehrere Speichereinrichtungen vorgesehen sein. Weiter alternativ kann die wenigstens eine Speichereinrichtung mit dem Möbel beziehungsweise mit der Warneinrichtung drahtgebunden oder alternativ drahtlos verbunden sein. Es ist auch möglich, eine hintereinander geschaltete Abfolge aus drahtgebunden und drahtlos gekoppelten Speichereinrichtungen einzusetzen, wenn beispielsweise eine räumlich vom Möbel getrennte Speichereinrichtung in Form einer Cloud Verwendung findet. Eine andere räumlich vom Möbel getrennte Speichereinrichtung kann Teil eines Möbelgerätes, z.B. eines Smartphones oder dergleichen sein.

Das Ausgeben eines Warnsignals erfolgt gemäß einer bevorzugten Ausführung bei einer hohen Übereinstimmung des wenigstens einen Bewegungs- und/oder Verhaltensmusters verglichen mit dem Vergleichsmuster. Da alle detektierte und analysierte Bewegungs- und/oder Verhaltensmuster naturgemäß jedoch geringfügig voneinander abweichen, kann ein Übereinstimmungsgrad berücksichtigt werden, wobei bei Erreichen oder Überschreiten des Übereinstimmungsgrades das Warnsignal ausgelöst wird. Der Übereinstimmungsgrad sagt aus, mit welcher Ähnlichkeit das Vergleichsmuster dem detektierten und analysierten Bewegungs- und/oder Vergleichsmuster entspricht.

Ferner kann der Übereinstimmungsgrad in vorgegebenen Grenzen verändert werden. Ebenso ist es möglich, den Grad des Einflusses einzelner Sensoren zur Bestimmung und zur Analyse des Bewegungs- und/oder Verhaltensmusters in vorgegebenen Grenzen verändern zu können. Sowie dieser Grad, als auch alternativ der Übereinstimmungsgrad und ebenso alternativ die Zeitdauer des vorherrschenden Bewegungs- und/oder Verhaltensmusters oder alternativ eine Zeitverzögerung zur verzögerten Ausgabe des Warnsignals kann mittels Software eingestellt werden. Die Einstellung erfolgt in einer beispielhaften Ausführung durch ein Smartphone, Tablet-Computer, Laptop oder dergleichen, wobei eine darauf lauffähige Software oder App zur Konfiguration und Einstellung solcher vorgenannten Parameter und Grade mit der Warneinrichtung kommunikativ verbindbar ausgebildet ist.

Geeignete Sensoren, um einen Bewegungs- und/oder Verhaltensmuster erfassen zu können, sind beispielsweise Kraftsensoren. Ein Kraftsensor ist dazu ausgebildet, prinzipiell eine Gewichtskraft oder eine Muskelkraft oder dergleichen erfassen zu können. Alternative Bauformen von Kraftsensoren werden zwar auch mit Gewichts- beziehungsweise Muskelkräften beaufschlagt, geben als elektrische Ausgangsgröße aber vielmehr ein quantitatives Signal aus, welches zum Bewegungs- und Verhaltensmuster korrespondiert. Bevorzugt werden mehrere Sensoren, die an verschiedenen Positionen im Ruhemöbel angeordnet sind, eingesetzt. Dabei ist erfindungsgemäß eine zeitliche Abfolge von Signalen, die von einem oder von verschiedenen Sensoren erfasst wurden, relevant. Die Kraftsensoren können als reine Drucksensoren oder als Beschleunigungs- bzw. Vibrationssensoren ausgebildet sein. Daneben kann ein Sensor auch als Mikrophon ausgebildet sein, um Luftschall zu erfassen und auszuwerten, da häufig ein Aufstehversuch von Geräuschen, die auf eine Anstrengung schließen lassen, begleitet ist.

Die Warneinrichtung kann zusätzlich mit mindestens einem weiteren Sensor gekoppelt sein, der extern vom Ruhemöbel angeordnet ist. Ein solcher externer Sensor kann beispielsweise ein Schallaufnehmer, z.B. ein Mikrophon, sein.

Weiterhin können alternativ noch folgende Sensoren vorgesehen sein: Lagesensoren, Wärmesensoren, Bewegungsmelder auf PIR-Basis (Pyroelectric Infrared Sensor - pyroelektrischer Sensor) oder auf der Basis passiver InfrarotSensoren (Passive Infrared Sensor), Sensoren zur Aufnahme von Körperschall). Weiter können Sensoren in Form von Piezosensoren, Widerstandssensoren, Kapazitive Sensoren zum Einsatz kommen. Alle der genannten Sensoren können einzeln oder auch in Kombination miteinander verwendet werden.

Die Warneinrichtung kann unmittelbar akustische oder optische Warnsignale abgeben. Alternativ oder zusätzlich ist es denkbar, dass Warnsignale in Form von Datensignalen abgegeben werden, beispielsweise über eine Bluetooth- oder WLAN (wireless local area network)-Verbindung. Derartige Warnsignale sind dann im Netzwerk verfügbar und können auch in benachbarten Räumen, beispielsweise über ein Smartphone oder einen Benachrichtigungspieper an das Pflegepersonal weiter gegeben werden.

Besonders bevorzugt sind verschiedene der genannten Sensoren in unterschiedlichen Elementen des Ruhemöbels angeordnet. Beispielsweise können Sensoren in einer Rückenlehne ebenso wie im Bereich einer Sitzfläche eines Sitzmöbels, sowie in den Armstützen angeordnet sein. Häufig haben Sitzmöbel auch eine Fußablage, die vor einem Aufstehen heruntergeklappt werden muss, was ebenfalls über entsprechende Sensoren erfasst werden kann.

Häufig sind Ruhemöbel der genannten Art mit einem elektromotorischen Möbelantrieb zur Verstellung des Ruhemöbels ausgestattet. Wenn dieses der Fall ist, können eingestellte Positionen verschiedener Elemente des Ruhemöbels, beispielsweise der Rückenlehne oder eines Fußteils oder eine Neigungseinstellung einer Sitzfläche, ebenfalls detektiert werden. Bestimmte Einstellungen, z.B. aktuell eingenommene Positionen von bewegbaren Möbelteilen, können als ein zusätzliches Indiz eines bevorstehenden Aufstehmanövers interpretiert werden. Erfindungsgemäß werden je nach Stellung des elektromotorischen Möbelantriebs unterschiedliche Vergleichsmuster zur Analyse des erfassten Bewegungs- und Verhaltensmusters herangezogen.

In einer alternativen Ausgestaltung ist die Warneinrichtung als Schalteinrichtung ausgebildet, derart, dass das Warnsignal in Form eines Schaltsignals ausgegeben wird. Beispielsweise kann die Warneinrichtung mit einem elektromotorischen Möbelantrieb gekoppelt sein. Ein solcher elektromotorischer Möbelantrieb dient der Bewegung wenigstens eines Möbelbauteils relativ zu einem weiteren Möbelbauteil. Das Bewegungs- und/oder Verhaltensmuster beziehungsweise der Vergleich dessen mit einem Vergleichsmuster wie eingangs näher beschrieben, veranlasst dann ein Verstellen eines Möbelbauteils relativ zu einem anderen Möbelbauteil.

Dabei kann vorgesehen sein, nach Auslösen des Warn- bzw. Schaltsignals mithilfe der Verstellung des Möbelbauteils einen Aufstehversuch zu unterstützen oder wahlweise zu erschweren. Beispielsweise kann durch das Anheben der Sitzfläche eine im Möbel ruhende Person angehoben werden, um ihr das Aufstehen zu erleichtern. Dies kann z.B. erfolgen, wenn das Bewegungs- und Verhaltensmuster einer im Möbel befindenden Person als beabsichtigtes Aufstehen erkannt wird.

Wenn mehrere Aktionen, z.B. das Ausgeben unterschiedlicher Warnsignale oder das Schalten verschiedener Verstellvorgänge, vorgesehen sind, kann eine Auswahlmöglichkeit für diese verschiedenen Aktionen vorgesehen sein. Eine solche Auswahl kann z.B. über eine entsprechende Bediensoftware oder einen oder mehrere Schalter vorgenommen werden. Als Bediensoftware kann ein Programm ("App") auf einem Smartphone oder Tablet-Computer dienen.

In einer alternativen Ausführungsform ist die Warneinrichtung als Nachrüstgerät ausgebildet, welches nachträglich mit einem bestehenden Möbel verbindbar ausgebildet ist.

In einer weiteren alternativen Ausführungsform ist die Warneinrichtung nicht unmittelbar am Ruhemöbel angeordnet, sondern mittelbar über einen Benutzer des Ruhemöbels. Beispielsweise trägt dann die ruhende Person die Warneinrichtung einschließlich des mindestens einen Sensors bevorzugt körpernah, z.B. in Form eines am Handgelenk oder am Gürtel oder an oder in einem Kleidungsstück vorgesehenen Geräts. Alternative kann ein solches Gerät der ruhenden Person umgehängt sein. Dabei entsprechen Aufbau und Wirkungsweise eines solchen Geräts, insbesondere im Hinblick auf das Erfassen der Sensorsignale, dem Erstellen und Analysieren des Bewegungs- und Verhaltensmusters und dem Ausgeben des Warnsignals der zuvor beschriebenen Anordnung von Warneinrichtung und des mindestens einen Sensors am Ruhemöbel, wobei die zuvor beschriebenen Ausführungen zumindest teilweise durch dieses als Mobilgerät ausgeführte Gerät verwirklicht sind. Sofern das Mobilgerät mit einer integrierten Sensorik, beispielsweise ein Beschleunigungssensor oder ein Mikrophon ausgestattet ist, entspricht die Beschreibung und Verwendung dieser Sensoren der eingangs genannten Beschreibung und Verwendung und soll an dieser Stelle nicht noch einmal wiederholt werden. Die Warneinrichtung ist nunmehr im Mobilgerät integriert, alternativ bildet das Mobilgerät die Warneinrichtung. Ist die Warneinrichtung im Mobilgerät integriert, so kann ein lauffähiges Programm ("App") des Mobilgeräts als Warneinrichtung verstanden werden. Solch ein Mobilgerät kann der Einfachheit halber als Smartphone ausgebildet sein.

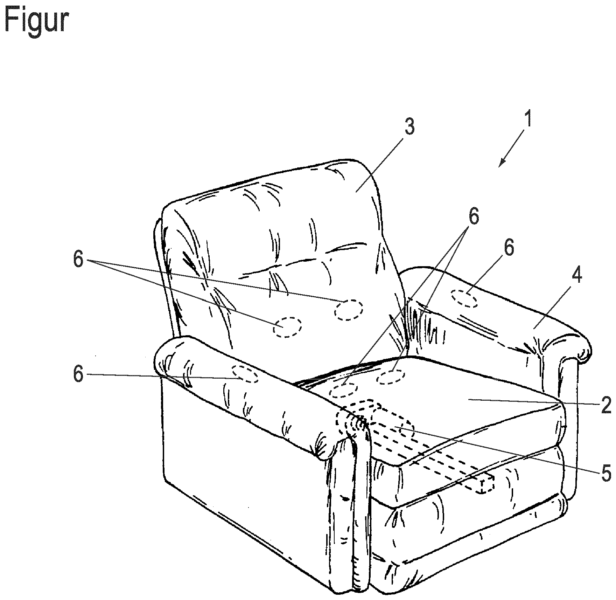

Die Erfindung wird nachfolgend anhand eines Ausführungsbeispiels mithilfe einer Figur näher erläutert. Die einzige Figur zeigt ein beispielhaftes Ruhemöbel mit einem elektromotorischen Möbelantrieb und einer Mehrzahl von Sensoren.

Beispielhaft ist in der Figur ein (Entspannungs-) Sessel 1, also ein Sitzmöbel, als Beispiel eines Ruhemöbels dargestellt. Der Sessel 1 umfasst eine Sitzfläche 2, eine Rückenlehne 3 und Armstützen 4. Gegebenenfalls kann ein ausfahrbares Fußteil vorgesehen sein, das im dargestellten Beispiel jedoch entweder nicht vorhanden ist oder eingefahren ist.

Der Sessel 1 weist weiter einen oder mehrere elektromotorische Möbelantriebe 5 auf, von denen einer gestrichelt eingezeichnet ist. Beispielsweise kann über den dargestellten elektromotorischen Möbelantrieb 5 eine Neigung der Rückenlehne 3 verstellt werden.

Der Sessel 1 weist eine hier nicht dargestellte Warneinrichtung auf, die mit einer Mehrzahl von Sensoren 6, die hinter einer Polsterung angeordnet sind, gekoppelt ist. Da wie vorliegend und erfindungsgemäß ein elektromotorischer Möbelantrieb 5 vorhanden ist, kann die Warneinrichtung in eine Steuereinrichtung des elektromotorischen Möbelantriebs integriert sein. Es können dann beispielsweise eine Stromversorgung und/oder eine Datenverbindung, die die Steuereinrichtung des elektromotorischen Möbelantriebs sowieso aufweist, von der Warneinrichtung mitbenutzt werden.

Beim dargestellten Beispiel sind sechs Sensoren 6 im Ruhemöbel angeordnet, zwei im Bereich der Sitzfläche 2, zwei in der Rückenlehne 3 und jeweils einer in jeder Armlehne 4. Die Sensoren 6 können Druck- bzw. Kraftsensoren sein oder auch Beschleunigungssensoren, über die ebenfalls eine auf dem entsprechenden Bereich einwirkende Kraft bzw. ein Kraftstoß beziehungsweise einer Bewegung detektiert werden kann.

Die Sensoren 6 werden von der Warneinrichtung erfasst und ausgewertet. Dabei kann beispielsweise ein zeitlich variierendes Aktivierungsmuster der Sensoren 6 aus den Sensordaten extrahiert werden und in einem Mustervergleich mit allgemeinen oder personenspezifischen hinterlegten Mustern verglichen werden. Deutet der Vergleich auf das Ausführen eines Bewegungs- und/oder Verhaltensmusters hin, das mit einem Aufstehvorgang verbunden ist oder diesen einleitet, kann die Warnvorrichtung ein entsprechendes Warnsignal unmittelbar oder über die zuvor genannten Datenverbindungen abgeben.

Dabei ist erfindungsgemäß vorgesehen, je nach Stellung des oder der elektromotorischen Möbelantriebe 5 unterschiedliche Vergleichsmuster und/oder Vergleichsschwellen zugrunde zu legen, da sich je nach Ausgangsanordnung der verschiedenen Komponenten des Ruhemöbels (z.B. Neigung der Sitzfläche 2 und der Rückenlehne 3) unterschiedliche Bewegungs- und Verhaltensmuster bei einem Aufstehvorgang oder dem Einleiten eines Aufstehvorgangs zu erwarten sind.

Der Mustervergleich kann dabei auf den Grundsätzen einer Fuzzy-Logik erfolgen, um einer natürlichen Schwankung, die auch ein wiederholter Bewegungsablauf zeigen wird, Rechnung zu tragen. Auch eine Auswertung in Form eines - gegebenenfalls Computer implementierten - neuronalen Netzes ist möglich.

### Bezugszeichenliste

- 1: (Entspannungs-) Sessel
- 2: Sitzfläche
- 3: Rückenlehne
- 4: Armstütze
- 5: elektromotorischer Möbelantrieb
- 6: Sensor

## Patentansprüche

1. Ruhemöbel mit wenigstens einem elektromotorischen Möbelantrieb zur Bewegung wenigstens eines Möbelbauteils relativ zu einem weiteren Möbelbauteil und mindestens einem Sensor (6) und einer mit diesem gekoppelten Warneinrichtung, um vor einem unerwünschten Aufstehen eines Benutzers des Ruhemöbels zu warnen, wobei die Warneinrichtung dazu eingerichtet ist, Informationen des mindestens einen Sensors (6) im Hinblick auf ein Bewegungs- und/oder Verhaltensmuster einer das Ruhemöbel benutzenden Person auszuwerten, um ein Warnsignal bereits bei einer Absicht, das Ruhemöbel zu verlassen, auszugeben, **dadurch gekennzeichnet, dass**
das Bewegungs- und/oder Verhaltensmuster durch eine zeitliche Abfolge von Signalen, die von einem oder von verschiedenen Sensoren (6) erfasst wurden, erstellt ist, und dass
die Warneinrichtung dazu eingerichtet ist, das erstellte Bewegungs- und/oder Verhaltensmuster zur Analyse mit vorgegebenen Vergleichsmustern zu vergleichen, wobei unterschiedliche Vergleichsmuster zur Analyse eingesetzt werden, abhängig von Stellungen des mindestens einen elektromotorischen Möbelantriebs, mit dem Elemente des Ruhemöbels verstellt werden können.

2. Ruhemöbel nach Anspruch 1, bei dem der mindestens eine Sensor (6) ein Kraftsensor, ein Drucksensor, ein Beschleunigungssensor und/oder ein Vibrationssensor ist.

3. Ruhemöbel nach Anspruch 1 oder 2, bei dem mehrere Sensoren (6) an dem Ruhemöbel angeordnet sind.

4. Ruhemöbel nach Anspruch 3, bei dem die mehreren Sensoren (6) auf verschiedene Komponenten des Ruhemöbels verteilt angeordnet sind.

5. Ruhemöbel nach einem der Ansprüche 1 bis 4, ausgebildet als Sitzmöbel und insbesondere als Sessel (1).

6. Ruhemöbel nach Anspruch 3 und 4, bei dem die mehreren Sensoren (6) im Bereich einer Sitzfläche (2), einer Rückenlehne (3), mindestens einer Armstütze (4) und/oder einem Fußteil angeordnet sind.

7. Ruhemöbel nach einem der Ansprüche 1 bis 6, bei dem die Warneinrichtung zusätzlich mit mindestens einem weiteren Sensor gekoppelt ist, der extern vom Ruhemöbel angeordnet ist.

8. Ruhemöbel nach Anspruch 7, bei dem der mindestens eine externe Sensor ein Schallaufnehmer ist.

9. Ruhemöbel nach einem der Ansprüche 1 bis 8, bei dem die Warneinrichtung dazu eingerichtet ist, ein akustisches und/oder ein optisches Warnsignal auszugeben.

10. Ruhemöbel nach einem der Ansprüche 1 bis 9, bei dem die Warneinrichtung dazu eingerichtet ist, ein Warnsignal als Datensignal auszugeben.

11. Verfahren zum Betreiben einer Warneinrichtung eines Ruhemöbels gemäß einem der Ansprüche 1 bis 10, die mit mindestens einem Sensor (6) gekoppelt ist, der am Ruhemöbel angeordnet ist, mit den folgenden Schritten:
- Erfassen von Signalen des mindestens einen Sensors (6);
- Auswerten der erfassten Signale;
- Erstellen eines Bewegungs- und/oder Verhaltensmusters einer das Ruhemöbel benutzenden Person anhand einer zeitlichen Abfolge der ausgewerteten Signale;
- Analysieren des Bewegungs- und/oder Verhaltensmusters und erkennen, ob eine Absicht, das Ruhemöbel zu verlassen, vorliegt, wobei das erstellte Bewegungs- und/oder Verhaltensmuster zur Analyse mit vorgegebenen Vergleichsmustern verglichen wird, wobei bei dem unterschiedliche Vergleichsmuster zur Analyse eingesetzt werden, abhängig von Stellungen des mindestens einen elektromotorischen Möbelantriebs, mit dem Elemente des Ruhemöbels verstellt werden können; und
- Ausgeben eines Warnsignals, wenn eine Absicht, das Ruhemöbel zu verlassen, erkannt wird.

12. Verfahren nach Anspruch 11, bei dem das Bewegungs- und/oder Verhaltensmuster anhand einer zeitlichen Abfolge von Signalen erstellt wird, die verschiedene Sensoren (6) erfasst haben.

13. Verfahren nach Anspruch 12, bei dem mindestens eines der vorgegebenen Vergleichsmuster aus einem der erstellten Bewegungs- und/oder Verhaltensmuster erzeugt ist.

## Claims

1. Reclining furniture comprising at least one electromotive furniture drive for moving at least one furniture component relative to another furniture component, and at least one sensor (6) and a warning device coupled thereto for warning of an undesirable rising of a user of the reclining furniture, wherein the warning device is designed to evaluate information of the at least one sensor (6) with respect to a movement and/or behavior pattern of a person using the reclining furniture in order to emit a warning signal already in the event of an intention to leave the reclining furniture, **characterized in that**
the movement and/or behavior pattern is created by a time sequence of signals detected by one or more sensors (6), and **in that**
the warning device motion is arranged to compare the created movement and/or behavior pattern with predetermined reference patterns for analysis, wherein different comparative patterns are used for analysis, depending on positions of the at least one electromotive furniture drive with which elements of the reclining furniture can be adjusted.

2. Reclining furniture according to claim 1, wherein the at least one sensor (6) is a force sensor, a pressure sensor, an acceleration sensor and/or a vibration sensor.

3. Reclining furniture according to claim 1 or 2, wherein a plurality of sensors (6) are arranged on the reclining furniture.

4. Reclining furniture according to claim 3, wherein the plurality of sensors (6) are distributed over various components of the reclining furniture.

5. Reclining furniture according to one of claims 1 to 4, designed as seating furniture and in particular as an armchair (1).

6. Reclining furniture according to claims 3 and 4, wherein the plurality of sensors (6) are arranged in the region of a seat surface (2), a backrest (3), at least one arm support (4) and/or a foot part.

7. Reclining furniture according to one of claims 1 to 6, wherein the warning device is additionally coupled to at least one further sensor which is arranged externally from the reclining furniture.

8. Reclining furniture according to claim 7, wherein at least one external sensor is a sound sensor.

9. Reclining furniture according to one of claims 1 to 8, wherein the warning device is designed to emit an audible and/or visual warning signal.

10. Reclining furniture according to one of claims 1 to 9, wherein the warning device is set up to output a warning signal as a data signal.

11. Method for operating a warning device of a reclining furniture according to one of claims 1 to 10, which is coupled to at least one sensor (6) arranged on the reclining furniture, having the following steps:
- acquiring signals from the at least one sensor (6);
- evaluating the acquired signals;
- creating a movement and/or behavior pattern of a person using the reclining furniture based a chronological order on the evaluated signals;
- analyzing the pattern of movement and/or behavior and determining whether there is any intention to leave the reclining furniture, wherein the motion and/or behavior pattern produced is compared with predetermined reference patterns for analysis and wherein different comparative patterns are used for analysis, depending on positions of the at least one electromotive furniture drive with which elements of the reclining furniture can be adjusted; and
- emitting a warning signal when an intention to leave the reclining furniture is detected.

12. Method according to claim 11, wherein the movement and/or behavior pattern is created on the basis of a temporal sequence of signals which have been detected by various sensors (6).

13. Method according to claim 12, wherein at least one of the predetermined patterns of comparison is generated from one of the created patterns of motion and/or behavior.

## Revendications

1. Meuble de repos avec au moins un entraînement à moteur électrique pour meuble pour le déplacement d'au moins un élément de meuble par rapport à un autre élément de meuble et au moins un capteur (6) et un dispositif d'avertissement relié à celui-ci pour avertir d'une intention importune de se lever d'un utilisateur du meuble de repos, le dispositif d'avertissement étant agencé pour évaluer les informations d'au moins un capteur (6) en rapport avec les modèles de mouvements et/ou de comportement d'une personne utilisant le meuble de repos afin d'émettre un signal d'avertissement déjà dans le cas d'une intention de quitter le meuble de repos, **caractérisé en ce que**
le modèle de mouvements et/ou de comportement est créé par une séquence temporelle de signaux détectés par un ou par différents capteurs (6) et que
le dispositif d'avertissement est agencé pour comparer le modèle de mouvements et/de comportement créé aux fins d'analyse avec les modèles de référence prédéfinis, différents modèles de référence étant utilisés aux fins d'analyse en fonction des positions d'au moins un entraînement à moteur électrique pour meuble, au moyen duquel des éléments du meuble de repos peuvent être déplacés.

2. Meuble de repos selon la revendication 1, dans lequel ledit au moins un capteur (6) est un capteur de force, un capteur de pression, un capteur d'accélération et/ou un capteur de vibrations.

3. Meuble de repos selon la revendication 1 ou 2, dans lequel plusieurs capteurs (6) sont disposés sur le meuble de repos.

4. Meuble de repos selon la revendication 3, dans lequel les différents capteurs (6) sont disposés répartis sur différents composants du meuble de repos.

5. Meuble de repos selon l'une des revendications de 1 à 4, conçu en tant que meuble pour s'asseoir, et notamment en tant que fauteuil (1).

6. Meuble de repos selon la revendication 3 ou 4, dans lequel les différents capteurs (6) sont disposés dans la zone d'une surface d'assise (2), d'un dossier (3), d'au moins un accoudoir (4) et/ou d'une partie de pied.

7. Meuble de repos selon l'une des revendications de 1 à 6, dans lequel le dispositif d'avertissement est relié en plus à un autre capteur disposé en dehors du meuble de repos.

8. Meuble de repos selon la revendication 7, dans lequel l'au moins un capteur externe est un capteur acoustique.

9. Meuble de repos selon l'une des revendications de 1 à 8, dans lequel le dispositif d'avertissement est agencé pour émettre un signal d'avertissement acoustique et/ou visuel.

10. Meuble de repos selon l'une des revendications de 1 à 9, dans lequel le dispositif d'avertissement est agencé pour émettre un signal d'avertissement en tant que signal de données.

11. Procédé pour faire fonctionner un dispositif d'avertissement d'un meuble de repos selon l'une des revendications de 1 à 10, qui est relié à au moins un capteur (6) disposé sur le meuble de repos, comportant les étapes suivantes :
- détection des signaux dudit au moins un capteur (6) ;
- évaluation de signaux détectés ;
- création d'un modèle de mouvements et/ou de comportement d'une personne utilisant le meuble de repos sur la base d'une séquence temporelle des signaux évalués ;
- analyse du modèle de mouvements et/ou de comportement et identification de la présence d'une intention de quitter le meuble de repos, le modèle de mouvements et/ou de comportement créé étant comparé aux modèles de référence prédéfini aux fins d'analyse, différents modèles de référence étant utilisés aux fins d'analyse en fonction des positions d'au moins un entraînement à moteur électrique pour meuble, au moyen duquel des éléments du meuble de repos peuvent être déplacés ; et
- émission d'un signal d'avertissement en cas d'identification d'une intention de quitter le meuble de repos.

12. Procédé selon la revendication 11, dans lequel le modèle de mouvements et/ou de comportement est créé sur la base d'une séquence temporelle des signaux détectés par différents capteurs (6).

13. Procédé selon la revendication 12, dans lequel au moins un des modèles de référence prédéfinis est généré à partir d'un des modèles de mouvements et/ou de comportement créés.
